(19) Europäisches Patentamt
European Patent Office
Office européen des brevets

(11) Veröffentlichungsnummer: 0 445 577 A2

(12) # EUROPÄISCHE PATENTANMELDUNG

(21) Anmeldenummer: **91102402.4**

(22) Anmeldetag: **20.02.91**

(51) Int. Cl.5: **C07D 519/00**, C07D 221/14, C08G 73/10, //(C07D519/00, 471:00,471:00)

(30) Priorität: **03.03.90 DE 4006646**

(43) Veröffentlichungstag der Anmeldung: **11.09.91 Patentblatt 91/37**

(84) Benannte Vertragsstaaten: **BE DE FR GB**

(71) Anmelder: **BASF Aktiengesellschaft**
**Carl-Bosch-Strasse 38**
**W-6700 Ludwigshafen(DE)**

(72) Erfinder: **Naarmann, Herbert, Dr.**
**Haardtblick 15**
**W-6719 Wattenheim(DE)**
Erfinder: **Cosmo, Robert, Dr.**
**O 4, 27**
**W-6800 Mannheim(DE)**

(54) **Polyperylen.**

(57) Polyperyleneinheiten der Formel I enthaltender Stoff

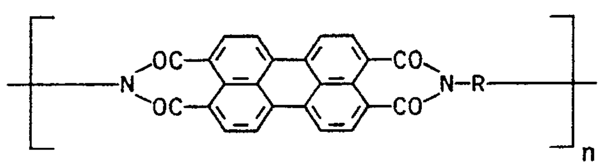

(I)

in der R einen aromatischen Rest und n eine Zahl bzw. einen Polymerisationsgrad von mehr als 1 bedeutet, wie er erhalten wird, wenn man ein entsprechendes Bis-naphthalinsäureimid der Formel II

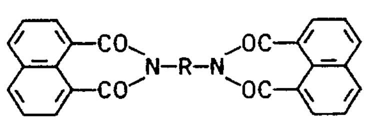

(II)

oxidiert.

EP 0 445 577 A2

Es ist bereits bekannt, niedermolekulare Perylen-Derivate z.B. durch Kondensation von Perylentetracarbonsäure mit Aminen herzustellen. Hierbei entstehen Perylenimide (A) bzw. Perylenimidazole (B).

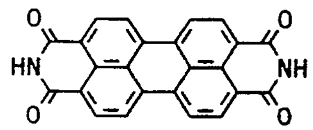

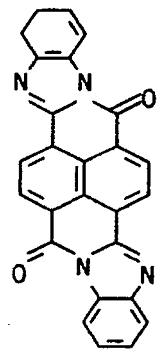

trans

+

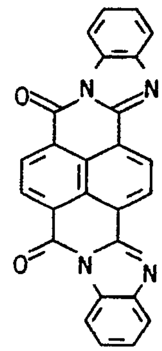

cis

(A)

(B)

Aufgabe der Erfindung ist es, ausgehend von löslichen Rohstoffen, polymere, d.h. hochmolekulare Perylenverbindungen herzustellen.

Die Lösung der Aufgabe wird dadurch möglich, daß es gelungen ist, 1,8-Naphthalinsäureanhydrid (III) mit geeigneten Diaminen des Typs $H_2N\text{-}R\text{-}NH_2$ (IV) zu dem entsprechenden Bis-1,8-Naphthalinsäureimid umzusetzen entsprechend dem nachstehenden Schema:

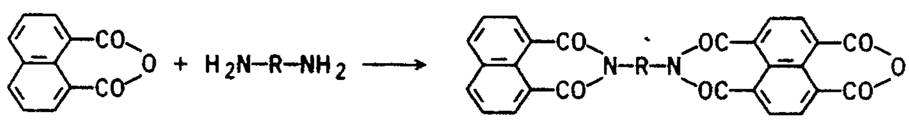

(III) (IV) (II)

Das erfindungsgemäße Bis-imid II wird dann erfindungsgemäß durch oxidative Kupplung in ein Polyperylen der Formel I überführt. Die Endgruppen weisen offenbar die Struktur des Naphthalindicarbonsäureimids auf.

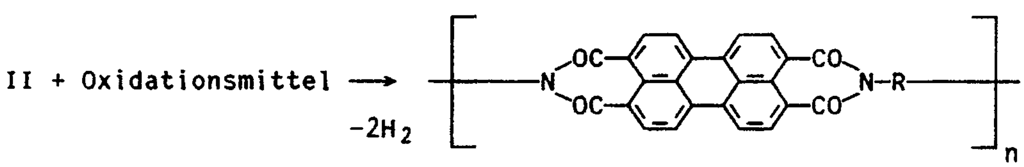


(I)

Das Diimid II erhält man in bekannter Weise (Thermally Stable Polymers, P.E. Cassidy p. 95-140, 1980, Mercel Dekker Inc. N.Y.), vorzugsweise durch Kondensation von 1,8-Naphthalinsäureanhydrid (IV) mit einem Diamin der Formel V in Gegenwart von wasserbindenden Agentien, wie Essigsäureanhydrid, Phosphorpentoxid, sirupöser Phosphoräure od. ä.. Als Diamin der Struktur V werden mehrkernige aromatische Verbindungen bevorzugt, in denen R jeweils einen zweiwertigen Rest, abgeleitet von den folgenden Kohlenwasserstoffen bzw. Aromaten, bedeutet.

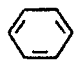, 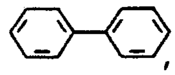, 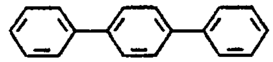

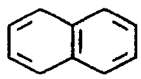, 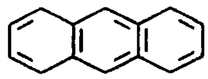

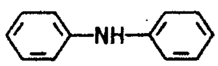, 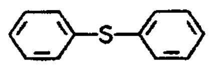, 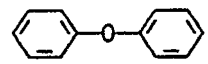

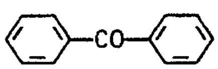, 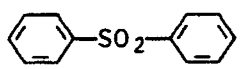, 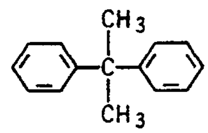

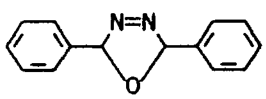 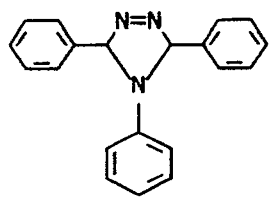

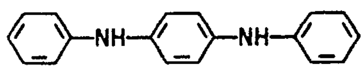 und seine O, S, CO, $SO_2$-Analogen

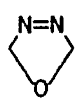, 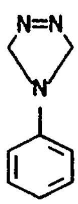

Die Verknüpfung ist in o-, m- oder p-Position.

Die aus Naphthalinsäureanhydrid und Diamin erhaltenen Bisimide II werden durch oxidative Kupplung in das Polyperylen I überführt.

Hierzu eignen sich anorganische oder organische Oxidationsmittel, deren Oxidationspotential größer als 0,5 Volt ist. Sie können unter neutralen, sauren oder basischen Bedingungen angewendet werden. Geeignet ist z.B. Luft, Sauerstoff, Arsensäure, Eisen-III-Verbindungen, Mangandioxid, Chromsäure, Bleidioxid, Permanganat, Silber-II-Verbindungen, Persulfat; außerdem ist die elektrochemische (anodische) Oxidation geeignet.

Oxidiert wird unter üblichen Bedingungen; bevorzugt sind Verfahren, bei denen eine innige Kontaktierung des Bis-1,8-Naphthalinsäureimids mit den jeweiligen Oxidationsmitteln erfolgen kann, z.B. die Umsetzung in der Schwingmühle, im Wirbelbett oder in Suspension mit indifferenten Hilfsflüssigkeiten. Die Oxidationsreaktion zur oxidativen Kupplung von II zu I muß offensichtlich mit äquivalenten Mengen des Oxidationsmittels durchgeführt werden. Die Reaktionstemperatur kann bis 500 °C betragen, vorzugsweise bis 150 °C.

Die elektrochemische Oxidation erfolgt anodisch vorzugsweise an einer Platin-, Silber- oder Bleielektrode mit einer Stromdichte von 5 $mA/cm^2$ bis 1000 $mA/cm^2$, vorzugsweise 10 bis 100 $mA/cm^2$; als Elektrolyt ist z.B. Propylencarbonat, Dioxan, Wasser, Glykoldimethylether oder Methylenchlorid geeignet und man verwendet ein Leitsalz, vorzugsweise Alkali- oder Ammoniumsalze von $PF_6^-$, $BF_4^-$, $ClO_4^-$. Das zu oxidierende Bis-1,8-Naphthalinsäureimid wird zweckmäßig in einem Netz auf die jeweilige Anode gepreßt.

Die nach dem Verfahren hergestellten Polyperylene sind in den üblichen Lösungsmitteln unlöslich und

als Pigmente, Sensoren, Elektroden und als Materialien zur Energiespeicherung geeignet.

Beispiel 1

19,8 g 1,8-Naphthalinsäureanhydrid werden mit 9,2 g p,p'-Diaminodiphenyl versetzt und nach Zugabe von 250 g sirupöser Phosphorsäure 8 Stunden unter Rühren auf 150° C erwärmt. Das Reaktionsgemisch wird noch heiß in 500 ml Wasser getropft, abgenutscht und dann mit kaltem Wasser (Eiswasser) säurefrei gewaschen.

Man trocknet bei 0,1 mbar während 8 Stunden bei 100° C und erhält 24 g des Produkts mit einem Schmelzpunkt (unter Zersetzung) von 345° C; die Reinheit ist etwa 95%.

Das IR-Spektrum zeigt eine Imid-Struktur (2 Banden 1745 und 1712 $cm^{-1}$), dagegen keine Amid-, -CONH- oder -COOH-Funktion.

| | | | |
|---|---|---|---|
| Die Elementaranalyse: | | theoret. | gef. |
| | | $C_{36}$: 79,4% | 79,9% |
| Molmasse: | 544 theoret. | $H_{20}$: 3,7 | 3,3 |
| | 560 gef. (osmometr.) | $N_4$: 11,8 | 11,4 |
| | | $O_2$: 5,1 | 5,2 |

Löslichkeit höchstens 1% in DMSO oder Trimethylpyrrolidin

Beispiele 2 bis 4

Entsprechend Beispiel 1 wurden die in der Tabelle aufgeführten Diamine eingesetzt und entsprechende Produkte erhalten.

| Nr. | Diamin Art und Menge (g) | Ausbeute Menge(g) | % | Elementaranalyse %* C | H | O | N | S | Fp. °C |
|---|---|---|---|---|---|---|---|---|---|
| | | | | (75,0) | (3,5) | (11,1) | (4,9) | (5,5) | |
| 2 | 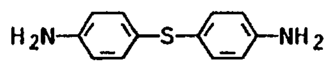 10,8 | 23,5 | 87,0 | 75,2 | 3,7 | 11,4 | 4,5 | 5,2 | 325 (Zers.) |
| | | | | (71,0) | (3,3) | (15,8) | (4,6) | (5,3) | |
| 3 | 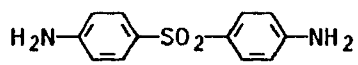 12,4 | 25,0 | 87,4 | 71,2 | 3,5 | 15,9 | 4,2 | 5,1 | 350 |
| | | | | (74,5) | (3,3) | (13,1) | (9,1) | - | |
| 4 | 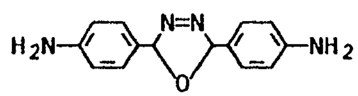 12,7 | 22,5 | 79,5 | 75,1 | 3,2 | 13,0 | 8,7 | - | 385 |

* Werte in Klammern sind Formelwerte

Beispiele 5 bis 10

Die in den Beispielen 1 bis 5 beschriebenen Bis-1,8-Naphthalinsäureidime II wurden durch oxidative Kupplung in die Polyperylenverbindungen I bzw. Ia überführt.

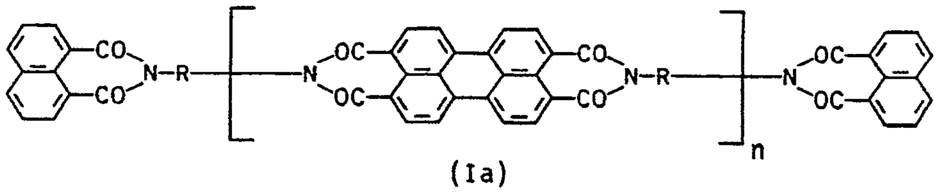

(Ia)

Bei der Kupplungsreaktion verbleiben als Endgruppen Naphthalinsäuremid-Reste (NSI), die mit den gebildeten difunktionellen Perylengruppen (CP) korrelieren, d.h. es gilt n(1) = NSI/P.

Die Bestimmung von NSI bzw. P erfolgt spektroskopisch mit Hilfe von IR und $C^{13}$ NMR.

Praktisch wurde so verfahren, daß z. B. 5,44 g des Bis-1,8-Naphthalinsäureimids(hergestellt nach Beispiel 1) bei 100° C im Zyklon-Wirbelbett einer Luftströmung von 1 m/sec für 10 Stunden ausgesetzt wurden. Die Luftzufuhr betrug 10 1/Stunde. Das gebildete Wasser wurde in einer Kältefalle (-76°) ausgefroren und gewogen. Das Wertprodukt wurde im Kreis geführt.

Ausbeute: 5,3 g eines unlöslichen Polyperylenderivates (Ia), dessen Kondensationsgrad n (entspr. Formel Ia) bei 12 liegt.

Das Polymere zersetzt sich oberhalb von 420° C.

Durch Zugabe von TCNQ (Tetracyanochinindimethan (0,01 Mol TCNQ + 0,01 Mol der Verbindung (Ia) suspendiert in 100 ml DMSO) entsteht eine schwarzglänzende Masse; nach dem Trocknen zeigt die Masse eine Leitfähigkeit von $2.10^{+1}$ S/cm.

Wird wie vorstehend beschrieben gearbeitet, jedoch die Einsatzstoffe sowie die Oxidationsmittel variiert, so werden die folgenden Ergebnisse erzielt.

| Nr. | Diimid Art und Menge (g) | | Ausbeute (g) | Fp. °C | n | Oxidationsmittel Art und Menge-Teile |
|---|---|---|---|---|---|---|
| 6 | entspr. Bsp. 2 | 5,8 | 5,3 | > 430 | 15 | $FeCl_3$* |
| 7 | entspr. Bsp. 3 | 6,1 | 5,9 | > 450 | 10 | $CuCl_2$ 6/$AlCl_3$ 6** |
| 8 | entspr. Bsp. 4 | 6,1 | 5,8 | > 450 | 15 | $CuCl_2$ 6/$AlBr_3$ 6** |
| 9 | entspr. Bsp. 1 | 5,4 | 5,2 | 435 (Zers.) | 20 | $FeBr_3$** |
| 10 | entspr. Bsp. 1 | 5,4 | 5,0 | 425 (Zers.) | 15 | $V_2O_5$ 10*** |

* Die Reaktion erfolgt bei 100°C , 5 Stunden in einer Schwingmühle, Aufarbeitung: Auskochen mit HCl, dann neutral waschen

** wie bei 6

*** wie bei 5; Versuche, das $V_2O_2$ bzw. andere gebildete V-Oxide abzutrennen waren vergeblich

## Patentansprüche

1. Polyperyleneinheiten der Formel I enthaltender Stoff

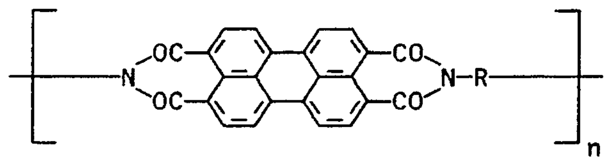

(I)

in der R einen aromatischen Rest und n eine Zahl bzw. einen Polymerisationsgrad von mehr als 1 bedeutet, wie er erhalten wird, wenn man ein entsprechendes Bis-naphthalinsäureimid der Formel II

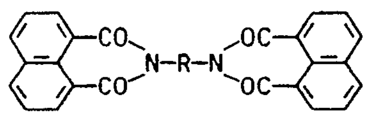

(II)

oxidiert.

**2.** Bis-naphthalinsäureimid der Formel II

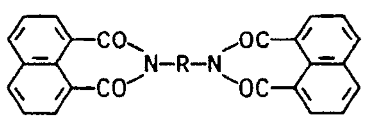

(II)

**3.** Verfahren zur Herstellung von Polyperylen der Formel I nach Anspruch 1, dadurch gekennzeichnet, daß die Oxidation mit Luftsauerstoff im Wirbelbett vorgenommmen wird.

**4.** Verfahren zur Herstellung von Polyperylen der Formel I nach Anspruch 1, dadurch gekennzeichnet, daß die Oxidation auf elektrochemischem Weg vorgenommen wird.

**5.** Verfahren zur Herstellung von Polyperylen der Formel I nach Anspruch 1, dadurch gekennzeichnet, daß die Oxidation mit einem anorganischen oder organischen Oxidationsmittel mit einem Oxidationspotential von mehr als +0,5 V vorgenommen wird.

**6.** Verfahren zur Herstellung von Bisnaphthalinsäureimid der Formel II, dadurch gekennzeichnet, daß man 1,8-Naphthalinsäureanhydrid (III) mit einem entsprechenden Diamin der Formel IV

$H_2N-R-NH_2$

umsetzt.